# EUROPEAN PATENT SPECIFICATION

(11) **EP 3 102 186 B1**
(45) Date of publication and mention of the grant of the patent: **27.01.2021**
(21) Application number: 15746248.2
(22) Date of filing: 04.02.2015
(51) Int. Cl.: A61K 9/14, A61K 9/16, A61K 31/445, A61K 9/50

(54) **DONEPEZIL COMPOSITIONS AND METHOD OF TREATING ALZHEIMER'S DISEASE**
DONEZEPILZUSAMMENSETZUNGEN UND VERFAHREN ZUR BEHANDLUNG VON MORBUS ALZHEIMER
COMPOSITIONS DE DONÉPÉZIL ET PROCÉDÉ DE TRAITEMENT DE LA MALADIE D'ALZHEIMER

(30) Priority: 04.02.2014 US 201461935596 P
(43) Date of publication of application: 14.12.2016
(73) Proprietor: Forest Laboratories Holdings Limited, Hamilton HM12 (BM); Dedhiya, Mahendra, G., Pomona, NY 10970 (US); Vermani, Kavita, Fremont, CA 94536 (US); Katdare, Ashok, Berkeley, CA 94708 (US)
(72) Inventor: DEDHIYA, Mahendra, G., Pomona, NY 10970 (US); VERMANI, Kavita, Fremont, CA 94536 (US); KATDARE, Ashok, Berkeley, CA 94708 (US)
(74) Representative: Hoffmann Eitle
(86) International application number: PCT/US2015/014404
(87) International publication number: WO 2015/120013

(56) References cited:
- WO-A1-2009/001146
- WO-A1-2011/127235
- WO-A1-2012/026902
- WO-A1-2013/005094
- WO-A2-2004/037234
- WO-A2-2005/065645
- CN-A- 102 309 465
- US-A1- 2009 269 401
- US-A1- 2011 251 239

## Description

### FIELD OF THE INVENTION

The present invention relates to compositions comprising donepezil or a pharmaceutically acceptable salt thereof (e.g., donepezil hydrochloride) alone or in combination with memantine or a pharmaceutically acceptable salt thereof (e.g., memantine hydrochloride) and methods for treating disorders (e.g. Alzheimer's disease) comprising administering donepezil or a pharmaceutically acceptable salt thereof (e.g., donepezil hydrochloride) alone or in combination with memantine or a pharmaceutically acceptable salt thereof (e.g., memantine hydrochloride).

### BACKGROUND OF THE INVENTION

Donepezil hydrochloride (Aricept®) is an acetylcholinesterasae inhibitor (AChEI) approved for the treatment of dementia of the Alzheimer's type in the United States and is available as 5 mg and 10 mg immediate release tablets, 23 mg sustained release tablets and as 5 mg and 10 mg orally disintegrating tablets.

Memantine (Namenda®) (1-amino-3,5-dimethyl adamantane), which is disclosed, e.g., in U.S. Pat. Nos. 4,122,193; 4,273,774; and 5,061,703, is a systemically-active uncompetitive NMDA receptor antagonist having low to moderate affinity for the receptor and strong voltage dependency and rapid blocking/unblocking kinetics. Memantine hydrochloride is approved for the treatment of moderate to severe dementia of the Alzheimer's type in the United States and is available as Namenda® (5 and 10 mg BID immediate release tablets) and Namenda XR® (28 mg once-daily extended release capsules).

There is an existing and continual need for formulations comprising donepezil that provide reliable delivery and absorption of the active ingredient, while also providing a dosing regimen that is straightforward and increases patient compliance. The present invention provides novel compositions and dosage forms comprising donepezil hydrochloride alone or in combination with a second active ingredient (e.g. , memantine hydrochloride or a pharmaceutically acceptable salt thereof) that can be sprinkled on food and thereby increase patient compliance.

WO 2009/001146 A1 describes a pharmaceutical composition for oral administration comprising donepezil or a pharmaceutically acceptable salt thereof, and an amount of pH dependent excipient as a taste masking agent to suppress the release of donepezil in the pH environment of the oral cavity and increase the release of donepezil in acidic environment.

WO 2005/065645 A2 describes donepezil formulations, including amorphous donepezil or pharmaceutically acceptable salts thereof; sustained-release formulations; and donepezil sprinkle formulations.

WO 2012/026902 A1 describes combinations comprising donepezil, memantine and ginkgo biloba extract or any pharmaceutically acceptable derivative thereof, and use of this type of dosage form in the treatment of Alzheimer's disease.

WO 2011/127235 A1 describes combination therapy for the treatment of Alzheimer's disease and Alzheimer's disease related dementia. The combination comprises memantine in an immediate or sustained release form and donepezil in a sustained release form.

WO 2004/037234 A2 describes a combination of a 1-aminocyclohexane derivative such as memantine or neramexane and an acetylcholinesterase inhibitor such as galantamine, tacrine, donepezil or rivastigmine.

### SUMMARY OF THE INVENTION

According to some embodiments, the present invention provides compositions comprising a therapeutically effective amount of donepezil or a pharmaceutically acceptable salt thereof (e.g., donepezil hydrochloride) for oral administration.

According to some embodiments, the present invention provides methods for treating Alzheimer's disease by administering to a patient in need thereof, a therapeutically effective amount of donepezil or a pharmaceutically acceptable salt thereof (e.g., donepezil hydrochloride) alone or in combination with memantine or a pharmaceutically acceptable salt thereof (e.g., memantine hydrochloride).

According to some embodiments, the present invention provides oral dosage forms comprising a composition comprising donepezil or a pharmaceutically acceptable salt thereof for use in a method of treating moderate to severe dementia of the Alzheimer's type, wherein the composition has an angle of repose of less than 40 degrees; wherein the dosage form comprises memantine or a pharmaceutically acceptable salt thereof; and wherein more than 60% of the donepezil or pharmaceutically acceptable salt thereof dissolves within 5 minutes of administration to the patient.

According to some embodiments, the present invention provides oral dosage forms comprising a composition comprising donepezil or a pharmaceutically acceptable salt thereof for use in a method of treating moderate to severe dementia of the Alzheimer's type, wherein the dosage form may be sprinkled on food and more than 80% of the donepezil or pharmaceutically acceptable salt thereof dissolves within 30 minutes of administration to the patient; wherein the dosage form comprises memantine or a pharmaceutically acceptable salt thereof.

According to some embodiments, the present invention provides oral dosage forms comprising a composition comprising donepezil or a pharmaceutically acceptable salt thereof for use in a method of treating moderate to severe dementia of the Alzheimer's type, wherein the dosage form has a volume of less than 0.70 ml and the composition has a drug loading of 10%; wherein the composition comprises 10 mg donepezil or a pharmaceutically acceptable salt thereof; wherein the dosage form comprises memantine or a pharmaceutically acceptable salt thereof; and wherein more than 60% of the donepezil or pharmaceutically acceptable salt thereof dissolves within 5 minutes of administration to the patient.

According to some embodiments, the present invention provides a donepezil HCl granular composition comprising an intragranular component and an extragranular component; wherein said intragranular component comprises 10% donepezil HCl, 60% lactose monohydrate, 22% microcrystalline cellulose, 7.2% corn starch, 0.3% magnesium stearate, 0.1% colloidal silicon dioxide; and wherein said extragranular component comprises 0.2% colloidal silicon dioxide and 0.2% magnesium stearate, wherein the percentages are relative to the granular composition.

### BRIEF DESCRIPTION OF THE DRAWINGS

Figure 1 shows Comparative Dissolution Profiles of donepezil granules filled in capsules 10 mg and Aricept tablets 10 mg.
Figure 2 shows Comparative Dissolution Profiles of Donepezil HCl Capsules (Prepared by Roller Compaction, Containing Lactose and MCC) and Aricept Tablets 10 mg.
Figure 3 shows Comparative Dissolution Profiles of Donepezil HC1 10 mg Capsules and Aricept Tablets in 0.1N HC1.
Figure 4 shows Comparative Dissolution Profiles of Donepezil HC1 10 mg Capsules and Aricept Tablets in pH 4.5 Phosphate Buffer.
Figure 5 shows Comparative Dissolution Profiles of Donepezil HC1 10 mg Capsules and Aricept Tablets in pH 6.8 Phosphate Buffer.
Figure 6 shows Comparative Dissolution Profiles of Donepezil HC1 10 mg Capsules at Different pH (0.1N HC1, pH 4.5 Phosphate Buffer and pH 6.8 Phosphate Buffers).

### DETAILED DESCRIPTION OF THE INVENTION

According to some embodiments, the present invention provides compositions comprising a therapeutically effective amount of donepezil or a pharmaceutically acceptable salt thereof (e.g., donepezil hydrochloride) for oral administration.

In some embodiments, the present invention provides compositions comprising a therapeutically effective amount of donepezil or a pharmaceutically acceptable salt thereof (e.g., donepezil hydrochloride) in combination with memantine or a pharmaceutically acceptable salt thereof (e.g., memantine hydrochloride) for oral administration.

In some embodiments, the present invention provides methods for treating Alzheimer's disease by administering to a patient in need thereof, a therapeutically effective amount of donepezil or a pharmaceutically acceptable salt thereof (e.g., donepezil hydrochloride) alone or in combination with memantine or a pharmaceutically acceptable salt thereof (e.g., memantine hydrochloride).

According to some embodiments, the present invention provides oral dosage forms comprising a composition comprising donepezil or a pharmaceutically acceptable salt thereof for use in a method of treating moderate to severe dementia of the Alzheimer's type, wherein the composition has an angle of repose of less than 40 degrees; wherein the dosage form comprises memantine or a pharmaceutically acceptable salt thereof; and wherein more than 60% of the donepezil or pharmaceutically acceptable salt thereof dissolves within 5 minutes of administration to the patient.

According to some embodiments, the present invention provides oral dosage forms comprising a composition comprising donepezil or a pharmaceutically acceptable salt thereof for use in a method of treating moderate to severe dementia of the Alzheimer's type, wherein the dosage form may be sprinkled on food and more than 80% of the donepezil or pharmaceutically acceptable salt thereof dissolves within 30 minutes of administration to the patient; wherein the dosage form comprises memantine or a pharmaceutically acceptable salt thereof.

According to some embodiments, the present invention provides oral dosage forms comprising a composition comprising donepezil or a pharmaceutically acceptable salt thereof for use in a method of treating moderate to severe dementia of the Alzheimer's type, wherein the dosage form has a volume of less than 0.70 ml and the composition has a drug loading of 10%; wherein the composition comprises 10 mg donepezil or a pharmaceutically acceptable salt thereof; wherein the dosage form comprises memantine or a pharmaceutically acceptable salt thereof; and wherein more than 60% of the donepezil or pharmaceutically acceptable salt thereof dissolves within 5 minutes of administration to the patient.

According to some embodiments, the present invention provides a donepezil HCl granular composition comprising an intragranular component and an extragranular component; wherein said intragranular component comprises 10% donepezil HCl, 60% lactose monohydrate, 22% microcrystalline cellulose, 7.2% corn starch, 0.3% magnesium stearate, 0.1% colloidal silicon dioxide; and wherein said extragranular component comprises 0.2% colloidal silicon dioxide and 0.2% magnesium stearate, wherein the percentages are relative to the granular composition.

The dosage forms of the invention may comprise a composition comprising donepezil as donepezil hydrochloride in an immediate release form (e.g., 5 or 10 mg). The dosage forms may also comprise a composition comprising donepezil as donepezil hydrochloride in a modified release form, e.g., as a 23 mg sustained release dosage.

The dosage forms of the invention may further comprise memantine or a pharmaceutically acceptable salt thereof, e.g., memantine hydrochloride. The memantine may be provided as an immediate release (e.g., 5, 10 or 20 mg) or modified release form (e.g., 7, 14, 21, or 28 mg). For example, in some embodiments the dosage forms may comprise 14 mg memantine or a pharmaceutically acceptable salt thereof. In other embodiments the dosage forms may comprise 28 mg memantine or a pharmaceutically acceptable salt thereof.

In exemplary embodiments, the present invention may comprise an immediate release component and a modified release component. For example, the present invention may comprise immediate release donepezil and modified release memantine. The amount of each component will depend on the active ingredient that is formulated as either an immediate or modified release component. In some examples, the dosage forms will comprise 10 mg donepezil as an immediate release form and 14 mg memantine as a modified release dosage form. In other examples, the dosage forms will comprise 10 mg donepezil as an immediate release form and 28 mg memantine as a modified release dosage form. In other examples, the dosage forms will comprise 23 mg donepezil as a modified release dosage form and 14 mg memantine as a modified release dosage form. In other examples, the dosage forms will comprise 23 mg donepezil as a modified release dosage form and 28 mg memantine as a modified release dosage form.

For example, the dosage forms comprising an immediate release component and a modified release component may include an amount of donepezil in the immediate release form of approximately 1% to 15% w/w of drug loading, preferably 5% to 15%. An immediate release donepezil content of about 10% w/w is particularly preferred. The composition of the invention may exhibit more than one peak in the plasma concentration/time curve in any one dosing interval depending on a particular active ingredient used, relative amounts of the IR and MR components, and the dissolution properties of the MR component. Thus, compositions may be achieved that have specific release profiles.

In some embodiments, the dosage forms may include an immediate release component and a modified release component that may include beads, granules or combinations thereof. Beads are dose proportional, *i*.*e*., the same proportions of beads of different types can be used for different doses without significantly altering the percent drug released over time. Different doses are obtained by using different amounts of beads. Beads also enable a variety of dissolution profiles by mixing one or more types of beads with different dissolution properties or using multi-layer coatings, as additional drug layering over a polymer layer and subsequent coatings to prepare unitary beads. In some embodiments, the dosage forms of the invention may include beads, granules or suspensions filled into capsules, compressed into tablets, or filled into sachets. One or more types of modified release beads can be mixed together and encapsulated, or used as a sprinkle on the subject's food. According to the invention, the oral solid dosage form may be any of these forms. Preferably, the dosage form is a capsule.

A suitable immediate release form of donepezil may simply be particles of donepezil admixed with soluble components for example, sugars (*e.g.,* sucrose, mannitol, *etc.*), polymers (*e.g.,* polyethylene glycol, hydroxypropyl cellulose, hydroxypropyl methyl cellulose, *etc.*), surfactants (*e.g.,* sodium lauryl sulphate, chremophor, tweens, spans, pluronics, and the like), insoluble glidant components (*e.g.,* microcrystalline cellulose, calcium phosphate, talc, fumed silica, and the like), coating material (examples of suitable coating materials are polyethylene glycol, hydroxypropyl methyl cellulose, wax, fatty acids, etc.), dispersions in suitable material (examples are wax, polymers, pharmaceutically acceptable oils, soluble agents, etc.) or combinations of the above.

The angle of repose may be used to characterize the flow properties of solids. Angle of repose is a characteristic related to interparticulate friction or resistance to movement between particles. The angle of repose is the constant, three-dimensional angle (relative to the horizontal base) assumed by a cone-like pile of material formed by any of several different methods. A variety of angle of repose test methods are described in the literature. The most common methods for determining the static angle of repose can be classified on the basis of the following two important experimental variables: (1) The height of the "funnel" through which the powder passes may be fixed relative to the base, or the height may be varied as the pile forms and (2) the base upon which the pile forms may be of fixed diameter or the diameter of the powder cone may be allowed to vary as the pile forms.

In addition to the above methods, the following variations have been used to some extent in the pharmaceutical literature:
*Drained angle of repose* is determined by allowing an excess quantity of material positioned above a fixed diameter base to "drain" from the container. Formation of a cone of powder on the fixed diameter base allows determination of the drained angle of repose.

*Dynamic angle of repose* is determined by filling a cylinder (with a clear, flat cover on one end) and rotating it at a specified speed. The dynamic angle of repose is the angle (relative to the horizontal) formed by the flowing powder. The internal angle of kinetic friction is defined by the plane separating those particles sliding down the top layer of the powder and those particles that are rotating with the drum (with roughened surface).

Although there is some variation in the qualitative description of powder flow using the angle of repose, much of the pharmaceutical literature appears to be consistent with the classification shown below.

| Flow Property | Angle of Repose (degrees) |
|---|---|
| Excellent | 25-10 |
| Good | 31-35 |
| Fair-aid not needed | 36-40 |
| Passable-may hang up | 41-45 |
| Poor-must agitate, vibrate | 46-55 |
| Very poor | 56-65 |
| Very, very poor | >66 |

### Experimental Considerations for Angle of Repose

In some embodiments, the present invention provides dosage forms wherein the angle of repose of the composition comprising donepezil or salt thereof (e.g., donepezil HC1) is less than about 40 degrees. In other embodiments, the angle of repose is less than about 35 degrees. In other embodiments, the angle of repose is less than about 30 degrees. In some exemplary embodiments, the present invention provides dosage forms wherein the angle of repose is between about 25 and about 40 degrees.

In some embodiments, the compositions release more than about 80% donepezil or a pharmaceutically acceptable salt thereof (e.g., donepezil hydrochloride) within 60 minutes upon entry in a use environment, e.g., administration to a patient in need thereof. For example, the compositions may release more than about 80% donepezil within about 10 minutes, about 15 minutes, about 30 minutes, about 45 minutes or about 60 minutes. The dissolution rate or release can be measured using the methods provided in FDA guidance for immediate release and modified release dosage forms.

In some embodiments, entry into a use environment includes but is not limited to contact of a formulation of the invention with the gastric or enteric fluids of a patient to whom it is administered, or with a fluid intended to simulate gastric fluid. For example, the use environment includes, but is not limited to dissolution media (e.g., pH 1.2-6.8) commonly used for testing the dissolution rate of compositions. In some embodiments, use environment refers to the stomach or other portion of the gastrointestinal tract intended as the site of major absorption locus. The donepezil or hydrochloride salt may be released in a dissolution medium with a pH ranging from about 1.2 to 6.8. In exemplary embodiments, a dissolution medium of pH 6.8 is employed to simulate intestinal fluid. In some embodiments, a dissolution medium of pH 4.5 is employed. In still other embodiments, a dissolution medium of pH 1.2 is employed to simulate gastric fluid. In some examples, the dissolution medium may be maintained at about 37°C±1°C. In exemplary embodiments, the compositions are immediate release and provide a dissolution rate of >80% donepezil after about 30 minutes at pH 1.2. In some examples, >80% of donepezil HC1 dissolves within 30 minutes using USP Apparatus I at 100 rpm in a volume of 900 mL in each of the following media: (1) simulated gastric fluid USP without enzymes; (2) a pH 4.5 buffer, and (3) a pH 6.8 simulated intestinal fluid USP without enzymes.

In some embodiments, the present invention provides dosage forms wherein more than 80% of the donepezil or pharmaceutically acceptable salt thereof dissolves within 30 minutes of administration to the patient. In some embodiments, more than 60% of the donepezil or pharmaceutically acceptable salt thereof dissolves within 5 minutes of administration to the patient. In exemplary embodiments, more than 70% of the donepezil or pharmaceutically acceptable salt thereof dissolves within 5 minutes of administration to the patient. In some embodiments, more than 30% of the donepezil or pharmaceutically acceptable salt thereof dissolves within 2.5 minutes of administration to the patient. In other embodiments, more than 20% of the donepezil or pharmaceutically acceptable salt thereof dissolves within 2.5 minutes of administration to the patient.

In some embodiments, the present invention provides dosage forms wherein the oral dosage form has a volume of less than 0.70 ml. In other embodiments, the oral dosage forms have a volume of less than 0.50 ml. In other embodiments, the oral dosage forms have a volume of less than 0.40 ml.

### Definitions

As used throughout, "Aricept" means donepezil hydrochloride tablets that are approved in the United States (Aricept®) for the treatment of dementia of the Alzheimer's type and are available as 5 mg and 10 mg immediate release dosage forms.

The term "pharmaceutically acceptable" means biologically or pharmacologically compatible for in vivo use in animals or humans, and preferably means approved by a regulatory agency of the Federal or a state government or listed in the U.S. Pharmacopeia or other generally recognized pharmacopeia for use in animals, and more particularly in humans.

An "effective amount" means the amount of a composition according to the invention that, when administered to a patient for treating a condition is sufficient to effect such treatment. The "effective amount" will vary depending on the active ingredient, the state or condition to be treated and its severity, and the age, weight, physical condition and responsiveness of the mammal to be treated.

The term "therapeutically effective" applied to dose or amount refers to that quantity of a compound or pharmaceutical composition that is sufficient to result in a desired activity upon administration to a mammal in need thereof.

A "subject or patient in need thereof" means a person that has been diagnosed with Alzheimer's disease. A patient in need thereof may be limited to a "stabilized patient" which means a patient currently stabilized on either memantine HCl (10 mg twice daily or 28 mg extended release once daily and/or donepezil HCl 10 mg.

The term "administering" to a patient means to provide as an oral dosage form whole or sprinkled over food, *e.g.,* on applesauce.

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviations, per practice in the art. Alternatively, "about" with respect to the compositions can mean plus or minus a range of up to 20%, preferably up to 10%, more preferably up to 5%. Alternatively, particularly with respect to biological systems or processes, the term can mean within an order of magnitude, preferably within 5-fold, and more preferably within 2-fold, of a value.

The following examples are merely illustrative of the present invention.

### Example 1

A blend containing donepezil HCl, Microcrystalline cellulose (MCC), and starch was prepared and lubricated with magnesium stearate. The lubricated blend was compacted using a roller compactor. An oscillating granulator with 0.8 mm screen was used for milling of the compacts. After milling, all granules were passed through mesh #30, blended with extragranular excipients and lubricated with magnesium stearate. The lubricated granular blend was filled in size 4 hard gelatin capsule shells using a laboratory scale automated scale encapsulating machine.

Table 1 provides examples of compositions prepared using MCC as the diluent.

**Table 1: Composition and Properties of Donepezil HCl Capsules Prepared by Roller Compaction**

| ***Batch Number*** | ***1086-135-10*** | ***1086-135-10A*** | ***1086-140-10*** |
|---|---|---|---|
| ***Components*** | ***mg*/*unit*** | ***mg*/*unit*** | ***mg*/*unit*** |
| **Intragranular** | | | |
| Donepezil HCl | 10 | 10 | 10 |
| Microcrystalline cellulose NF (Avicel PH102) | 82.3 | 82.3 | 82.1 |
| Corn starch NF | 5 | 5 | 5 |
| Magnesium stearate NF | 0.25 | 0.25 | 0.25 |
| Colloidal silicon dioxide NF (Aerosil 200P) | 0.1 | 0.1 | 0.1 |
| **Extragranular** | | | |
| Corn starch NF | 2 | 2 | 2 |
| Magnesium stearate NF | 0.25 | 0.25 | 0.25 |
| Colloidal silicon dioxide NF (Aerosil 200P) | 0.1 | 0.4 | 0.3 |
| Total unit weight for 10 mg capsule | 100 mg | 100 mg | 100 mg |
| Batch size | 500 capsules | 500 capsules | 5000 capsules |
| **Characteristics** | | | |
| Angle of repose | ND | 30.96 | 29.93 |
| Bulk density (g/mL) | 0.48 | 0.48 | 0.47 |
| Tap density (g/mL) | 0.64 | 0.64 | 0.63 |
| Compressibility (t-b)/t | 25 | 25 | 25 |
| Hausener ratio (Tap/Bulk) | 1.33 | 1.33 | 1.34 |

The bulk density of donepezil granules of Batch 1086-135-10 is 0.48 g/mL, but the granules did not flow through the funnel during angle of repose measurements. Hence, colloidal silicon dioxide was also added extragranularly (#1086-135-10A) to the granules to improve the flow. A slightly larger batch (Batch 1086-140-10) was made and granules were encapsulated in size 4 capsule shells. Weight variation of capsules of this batch was found within acceptable range of ±10%.

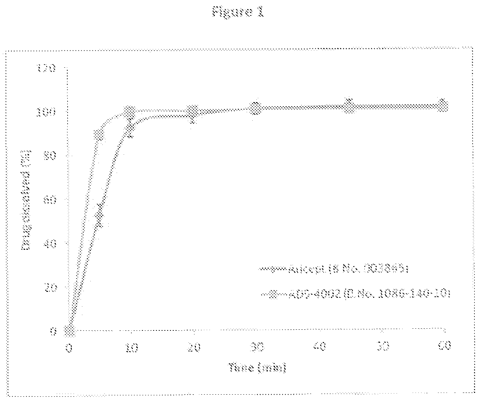

Dissolution profile of donepezil granules filled in capsules 10 mg (Batch 1086-140-10) was compared with Aricept tablets 10 mg. *See* Figure 1.

Tables 2 and 3 show the properties for donepezil Drug Substance and Aricept 10 mg Pulverized Tablets respectively.

**Table 2: Donepezil Drug Substance**

| | |
|---|---|
| Angle of repose (requires tapping) | >50.2 |
| Bulk density (g/mL) | 0.14 |
| Tap density (g/mL) | 0.46 |
| Compressibility (t-b)/t | 70 |
| Hausener ratio (Tap/Bulk) | 3.29 |

**Table 3: Aricept 10 mg Tablet Pulverized to powder in a mortar and pastel**

| | |
|---|---|
| Angle of repose | 43 |
| Bulk density (g/mL) | 0.626 |
| Tap density (g/mL) | 0.745 |
| Compressibility (t-b)/t | 16 |
| Hausener ratio (Tap/Bulk) | 1.19 |

### Example 2

A blend containing donepezil HCl, Microcrystalline cellulose (MCC), lactose monohydrate, and starch was prepared and lubricated with magnesium stearate. The lubricated blend was compacted using a roller compactor equipped with corrugated rollers at 2 ton roll pressure. An oscillating granulator with 0.8 mm screen was used for milling of the compacts. After milling, all granules were passed through mesh #30, blended with extragranular excipients and lubricated with magnesium stearate. The lubricated granular blend was filled in size 5 hard gelatin capsule shells using a laboratory automated scale encapsulating machine.

In the roller compaction experiments, different ratios of lactose monohydrate and MCC were evaluated as follows: 60:22.3 (Batch 1086-149-10) and 22.3:60 (Batch 1086 150 10). In both these experiments, a portion of lactose was also added in the extra granular portion. The bulk density of the blend with higher percentage of lactose (Batch 1086-149-10, 0.49 g/mL) was higher than the granules with lower amount of lactose (Batch 1086-150-10, 0.44 g/mL). Based on these observations, it was decided to use a higher percentage of lactose, all in intragranular portion and Batch 1086-172-10 was manufactured. A small amount of colloidal silicon dioxide was also added intragranularly to improve the flow of blend during roller compaction. A larger batch of granules (#1086-183-10) was manufactured and encapsulated in a smaller (size 5) capsule. Filling in smaller size capsules was possible because of the higher bulk density (0.6 g/mL). Weight variation of capsules was found within acceptable range of ±10%.

Table 4 provides Composition and Properties of Donepezil HCl Capsules Trial Batches (Prepared by Roller Compaction, Containing Lactose and MCC).

**Table 4: Composition and Properties of Donepezil HCl Capsules Trial Batches**

| ***Batch Number*** | ***1086-149-10*** | ***1086-150-10*** | ***1086-172-10*** | ***1086-183-10*** |
|---|---|---|---|---|
| ***Components*** | ***mg*/*unit*** | ***mg*/*unit*** | ***mg*/*unit*** | ***mg*/*unit*** |
| Intragranular | | | | |
| Donepezil HCl | 10 | 10 | 10 | 10 |
| Lactose monohydrate NF (Supertab 11SD) | 50 | 12.3 | 60 | 60 |
| Microcrystalline cellulose NF (Avicel PH102) | 22.3 | 60 | 22.0 | 22.0 |
| Corn starch NF | 7.4 | 7.4 | 7.2 | 7.2 |
| Magnesium stearate NF | 0.1 | 0.1 | 0.3 | 0.3 |
| Colloidal silicon dioxide NF (Aerosil 200P) | - | - | 0.1 | 0.1 |
| Extragranular | | | | |
| Lactose monohydrate NF (Supertab 11SD) | 10 | 10 | - | - |
| Magnesium stearate NF | 0.2 | 0.2 | 0.2 | 0.2 |
| Colloidal silicon dioxide NF (Aerosil 200P) | 0.2 | 0.1 | 0.2 | 0.2 |
| Total unit weight for 10 mg capsule | 100 mg | 100 mg | 100 mg | 100 mg |
| Batch size | 500 Capsules | 500 Capsules | 1000 Capsules | 8000 Capsules |
| Characteristics | | | | |
| Angle of repose | - | - | 34.95 | 32.96 |
| Bulk density (g/mL) | 0.49 | 0.44 | 0.52 | 0.60 |
| Tap density (g/mL) | 0.70 | 0.61 | 0.73 | 0.81 |

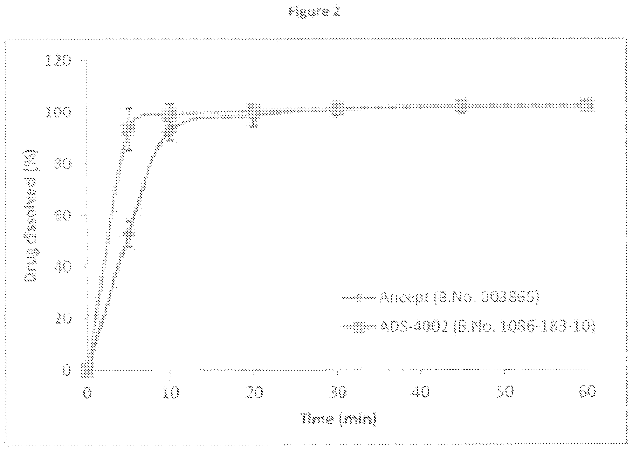

Dissolution profile of donepezil capsules 10 mg of Batch 1086 183-10 was compared with Aricept tablets 10 mg. *See* Figure 2. These granules also had a higher bulk density (0.6 g/mL) and good flow. Hence, the composition of this batch (1086 183 10) was considered suitable for the combination product (Memantine HCl ER/Donepezil HCl IR Capsules).

A GMP batch of selected formulation of donepezil HCl capsules 10 mg (1086-194-10) was manufactured at laboratory scale (8000 capsules) and Size 5 hard gelatin capsules of white color were used for a clinical study.

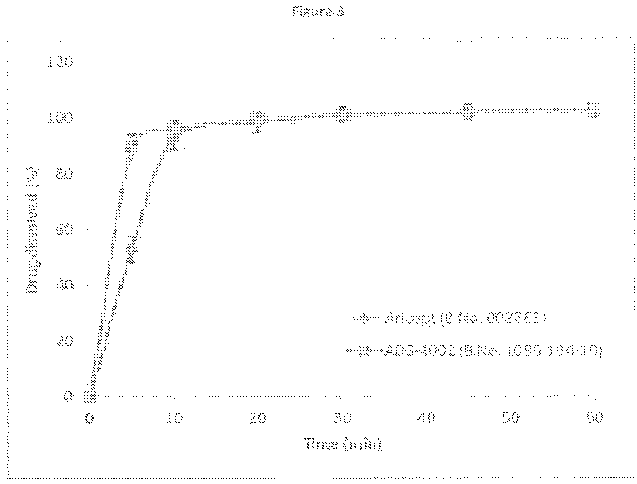

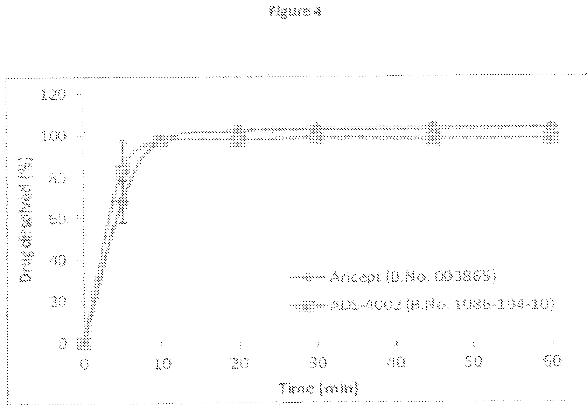

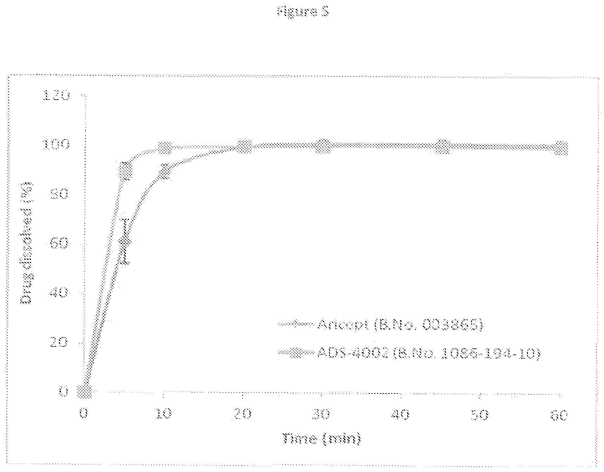

The dissolution of capsules of batch # 1086-194-10 were studied as a function of pH using different media i.e., a) 0.1N HCl, b) phosphate buffer (pH 4.5), and c) phosphate buffer (pH=6.8) and compared with Aricept tablets 10 mg (Batch 003865). *See* Figures 3-5 for the 0.1N HCl, pH 4.5, and pH 6.8 dissolution media, respectively.

The capsule formulation shows a faster initial release of the drug relative to Aricept and then levels off at about 10 minutes. This fast release from capsules shells is attributed to faster disintegration of capsule shells as compared to tablets. The tablet formulation (Aricept) shows a marginally slower initial release and levels off at about 30 minutes. In all instances, quantitative release is seen for all the lots evaluated at all three pH values evaluated.

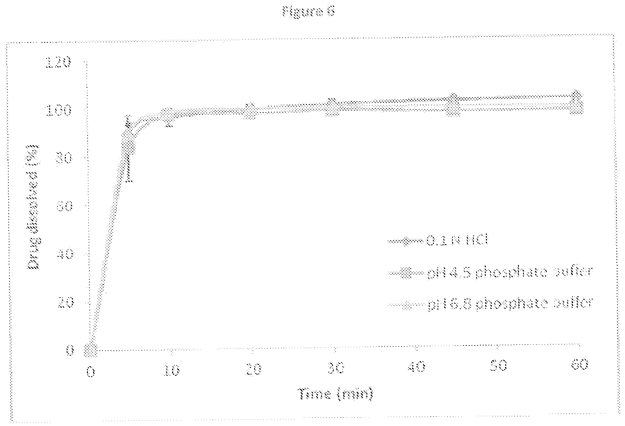

Figure 6 shows the dissolution profile of a formulation for a BE study using donepezil HCl capsules (Batch 1086-194-10) in media of different pH. The dissolution profile of donepezil HCl capsules was found to be similar in all the media studied.

### Example 3

Memantine HCl ER/Donepezil HCl capsules were developed as oral capsules containing a fixed dose combination of memantine hydrochloride (HCl) extended release (ER) beads and donepezil HCl immediate release (IR) granules for the treatment of moderate to severe dementia of the Alzheimer's type.

The quantitative composition donepezil HCl granules along with the grade, function, amount per capsule, and percent for each component are shown in Table 5.

**Table 5: Composition of Donepezil HCl Granules**

| ***Component*** | ***Function*** | ***% w*/*w of total granules*** | ***Amount per capsule (mg*/*unit)*** |
|---|---|---|---|
| Intragranular | | | |
| Donepezil Hydrochloride USP | Active | 10% | 10 |
| Lactose monohydrate NF (Supertab SD11) | Diluent | 60% | 60 |
| Microcrystalline cellulose NF (Avicel PH102) | Diluent | 22% | 22 |
| Corn starch NF | Disintegrant | 7.2% | 7.2 |
| Magnesium stearate NF | Lubricant | 0.3% | 0.3 |
| Colloidal silicon dioxide NF (Aerosil 200P) | Glidant | 0.1% | 0.1 |
| Extra granular | | | |
| Colloidal silicon dioxide NF (Aerosil 200P) | Glidant | 0.2% | 0.2 |
| Magnesium stearate NF | Lubricant | 0.2% | 0.2 |
| Total fill weight of donepezil granules | - | 100% | 100 |

| | | | |
|---|---|---|---|
| NF = National Formulary. | | | |

An immediate release composition of donepezil HCl granules with desired dissolution and density was developed in order to combine with memantine hydrochloride ER beads, in a small size capsule.

For convenient oral administration, size of the dosage form is important to make it easy to swallow. Tablet weight for Aricept® 10 mg tablets is 280 mg containing approximately 3.57% of drug loading. A similar drug loading in the granules was expected to result in high capsule fill weights that could only be accommodated in larger capsule sizes. A large capsule size was deemed undesirable from patient convenience perspective. To facilitate the encapsulation of two drug components in a small capsule, a higher drug loading and denser formulation of donepezil HCl was desired. Therefore, a drug loading of 10% w/w donepezil HCl was considered. In addition to Memantine HCl ER/Donepezil HCl capsules of strengths, 28/10 and 14/10, the strengths of 28/5 mg and 14/5 mg were contemplated. Drug loading of 10% w/w was expected to result in the fill weights of 100 mg and 50 mg respectively for 10 mg and 5 mg strengths. Drug loading higher than 10% w/w will result in lower than 50 mg fill weight for the lower strength, which might compromise fill accuracy using commonly available encapsulation machines. Hence, it was decided to develop donepezil blend/granule formulation with 10% w/w drug content.

Excipients for donepezil HCl granules were selected based on their chemical/physical compatibility with donepezil, regulatory acceptance, and processability. A drug excipient compatibility study was performed before selecting the final composition. Only excipients that were chemically compatible with donepezil HCl were used in the formulation of donepezil HCl granules. Most of the excipients selected for donepezil granulation of donepezil HCl granules are similar to those present in Aricept tablets 10 mg. To improve the density of composition, it was decided to use dry granulation by roller compaction as described above.

The donepezil HCl granules have higher drug loading (10% w/w) with immediate release characteristics. The dissolution profile in different pH was comparable to Aricept tablets. It is bioequivalent to Aricept tablets and stable for more than 24 months at room temperature.

Memantine HCl ER/Donepezil HCl capsules were developed by combining the Memantine HCl ER beads and Donepezil HCl granules (14/10 mg and 28/10 mg). Both memantine strengths are dose-proportional in that they are manufactured from a common ER beads, differing only in the filled amount of ER beads. Memantine HCl ER bead formulation was developed since it offered flexibility in achieving the desired memantine strengths using a common bead formulation.

The memantine HCl ER beads are multi-layered, consisting of sugar spheres which are layered with an aqueous dispersion of the API, talc, and povidone K30 to form drug layered beads. The drug layered beads are coated with Surelease® Clear dispersion to form polymer coated beads. The polymer coated beads are seal coated with Opadry® Clear. The excipients used in the drug product ER capsule formulation were selected based on their compatibility with the memantine HCl API.

### Example 4

A Single-Center, Randomized, Open-Label, 3-Way Crossover, Single-Dose Study was conducted to evaluate the effect of food and the effect of administration of capsule contents sprinkled on applesauce in healthy adults.

The approved Namenda XR doses are 7, 14, 21, and 28 mg. The approved Aricept strengths are 5, 10, and 23 mg; the 23-mg once-daily dose can only be administered once subjects have been on a dose of 10 mg once daily for at least 3 months. The proposed highest-dose strength for MDX-8704 is 28 mg memantine HCl ER/10 mg donepezil HCl. In this food-effect and relative bioavailability study, the MDX-8704 28 mg memantine HCl ER/10 mg donepezil HCl capsule was used in accordance with the FDA Guidance for Industry Food-Effect Bioavailability and Fed Bioequivalence Studies (US Food and Drug Administration, 2002) to conduct the study with the highest-dose strength.

A total of 36 healthy male and female subjects aged 18 through 45 years, were enrolled in the study. All 36 subjects were included in the safety analysis. Twenty-three subjects were included for the pharmacokinetic (PK) analysis of Treatment A (MDX-8704 intact capsule under fasted conditions), 23 for Treatment B (MDX-8704 intact capsule under fed conditions), and 21 for Treatment C (MDX-8704 capsule contents sprinkled on applesauce under fasted conditions).

Subjects were randomly assigned to 1 of 6 treatment sequences (ABC, ACB, BAC, BCA, CAB, or CBA).

**Table 6: Treatment sequences**

| | ***Period 1*** | ***Period 2*** | ***Period 3*** |
|---|---|---|---|
| Sequence I | Treatment A | Treatment B | Treatment C |
| Sequence II | Treatment A | Treatment C | Treatment B |
| Sequence III | Treatment B | Treatment A | Treatment C |
| Sequence IV | Treatment B | Treatment C | Treatment A |
| Sequence V | Treatment C | Treatment A | Treatment B |
| Sequence VI | Treatment C | Treatment B | Treatment A |

Each of the following treatments was administered with a 21-day washout period between treatments:
Treatment A: A single oral dose of MDX-8704 (memantine HCl ER/donepezil HCl) 28 mg/10 mg capsule administered under fasted conditions;
Treatment B: A single oral dose of MDX-8704 (memantine HCl ER/donepezil HCl) 28 mg/10 mg capsule administered following a high-fat breakfast; and
Treatment C: A single oral dose of MDX-8704 (memantine HCl ER/donepezil HCl) 28 mg/10 mg administered as capsule contents sprinkled on 30 mL (2 tablespoons) of applesauce under fasted conditions.

Blood samples were collected starting on Day 1 of each period at 0 hour (predose) and at 1, 2, 3, 4, 6, 8, 10, 12, 14, 24, 30, 36, 48, 72, 96, 120, 168, 216, and 264 hours after dosing. Plasma samples were analyzed for memantine and donepezil concentrations using a validated liquid chromatography coupled with tandem mass spectrometry method with good accuracy, linearity, reproducibility, and precision.

### Statistical Methodology:

Descriptive statistics for all PK parameters of memantine and donepezil were provided by treatment for all subjects who completed the study, had no episode of emesis (within 24 hours after administration of MDX-8704 for PK analysis of memantine and within 2 times the median Tmax [time of maximum plasma drug concentration] after administration of MDX-8704 for PK analysis of donepezil), and had evaluable PK parameters. The Cmax (maximum plasma drug concentration), AUC0-t (area under the plasma concentration versus time curve from time 0 to time t), and AUC0-∞ (area under the plasma concentration versus time curve from time 0 to infinity) parameters of memantine and donepezil were compared by means of a linear mixed effects model with sequence, treatment, and period as fixed effects and subjects within sequence as a random effect. Confidence intervals (CIs) of 90% were constructed for the ratio of geometric least squares (LS) means of Cmax, AUC0-t, and AUC0-∞ between MDX-8704 intact capsule under fed conditions (Treatment B) and MDX-8704 intact capsule under fasted conditions (Treatment A), and between MDX-8704 capsule contents sprinkled on applesauce under fasted conditions (Treatment C) and Treatment A. No food effect would be concluded if the corresponding 90% CIs of the ratio of geometric LS means of Cmax, AUC0-t, and AUC0-∞ between Treatment B and Treatment A for memantine and donepezil were within the range of 0.800 to 1.250. No difference between administrations of MDX-8704 as intact capsule versus capsule contents sprinkled on applesauce would be concluded if the corresponding 90% CIs of the ratio of geometric LS means of Cmax, AUC0-t, and AUC0-∞ between Treatment C and Treatment A for memantine and donepezil were within the range of 0.800 to 1.250. Arithmetic mean ratios of Tmax for memantine and donepezil were presented between Treatment B and Treatment A, and between Treatment C and Treatment A. The Wilcoxon signed-rank test was performed on Tmax. A p-value ≤0.05 was considered a significant difference between treatments.

Safety parameters (adverse events [AEs], vital sign assessments, clinical laboratory evaluations, electrocardiographic [ECG] parameters, physical examination findings, and results of the Columbia-Suicide Severity Rating Scale [C-SSRS] were summarized for all subjects who took at least 1 dose of investigational product.

### Results:

Subject Disposition: Thirty-six subjects were enrolled in the study. Three subjects (8.33%) prematurely discontinued from the study. One subject (2.78%) discontinued due to an AE; 1 subject (2.78%) discontinued due to a protocol violation, and 1 subject (2.78%) discontinued for other reasons.

For subjects in the Safety Population, the mean age (± SD) and body mass index (± SD) were 27.3 (± 5.3) years and 25.41 (± 2.90) kg/m2, respectively. Overall, 25 subjects (69.44%) were male and 11 subjects (30.56%) were female.

Pharmacokinetics: The PK Population consisted of all subjects who completed the study and had evaluable PK parameters for Treatments A and B (intact capsule administered with and without food) or Treatments A and C (intact capsule versus capsule contents sprinkled on applesauce under fasted conditions). Subjects who vomited within 24 hours after administration of MDX-8704 were excluded from the PK analysis of memantine. Subjects who vomited within 2 times the median Tmax after administration of MDX-8704 were excluded from the PK analysis of donepezil. Data from subjects in the PK Population were used for PK analysis and summary statistics of PK parameters.

Twenty-three subjects were included in the food effect analysis (intact capsule administered with and without food) of memantine and donepezil; 21 subjects were included in the comparison of administration of intact capsule under fasted conditions versus capsule contents sprinkled on applesauce under fasted conditions for both memantine and donepezil.

The 90% CIs for the geometric LS means ratios of Cmax, AUC0-t, and AUC0-∞ for memantine in the comparison of MDX-8704 intact capsule under fed conditions (Treatment B) versus administration of MDX-8704 intact capsule under fasted conditions (Treatment A) were within the range of 0.800 to 1.250, indicating no significant food effect. In addition, the 90% CIs for the geometric LS means ratios of Cmax, AUC0-t, and AUC0-∞ for memantine in the comparison of MDX-8704 capsule contents sprinkled on applesauce under fasted conditions (Treatment C) versus Treatment A were within the range of 0.800 to 1.250, indicating no statistically significant difference between administration of MDX-8704 as intact capsule under fasted conditions versus capsule contents sprinkled on applesauce under fasted conditions. The mean terminal elimination half-life of memantine was observed to be 62.07, 59.75, and 62.77 hours for Treatments A, B, and C, respectively. There was a statistically significant difference in the median Tmax for memantine following administration of Treatment A versus Treatment B with a p-value of 0.014; and following administration of Treatment A versus Treatment C with a p-value of 0.012.

The 90% CIs for the geometric LS means ratios of Cmax, AUC0-t, and AUC0-∞ for donepezil in the comparison of MDX-8704 intact capsule under fed conditions (Treatment B) versus administration of MDX-8704 intact capsule under fasted conditions (Treatment A) were within the range of 0.800 to 1.250, indicating no significant food effect. In addition, the 90% CIs for the geometric LS means ratios of Cmax, AUC0-t, and AUC0-∞ for donepezil in the comparison of MDX-8704 capsule contents sprinkled on applesauce under fasted conditions (Treatment C) versus Treatment A were within the range of 0.800 to 1.250, indicating no statistically significant difference between administration of MDX-8704 as intact capsule under fasted conditions versus capsule contents sprinkled on applesauce under fasted conditions. The mean terminal elimination half-life of donepezil was observed to be 67.26, 66.62, and 65.01 for Treatments A, B, and C, respectively. There was a statistically significant difference in the median Tmax for donepezil following administration of Treatment A versus Treatment B with a p-value of < 0.001. There was no statistically significant difference between the median Tmax for donepezil following administration of Treatment A versus Treatment C with a p-value of 0.278.

There was no statistically significant difference in the total exposure and peak concentration of memantine and donepezil following administration of MDX-8704 (memantine ER/donepezil) 28 mg/10 mg capsule with a high-fat meal versus under fasted conditions, suggesting food has no significant effect on the bioavailability of the MDX-8704 capsule. The median Tmax was reduced to 14.0 hours from 24.0 hours for memantine, but was prolonged to 6.0 hours from 3.0 hours for donepezil when administered with the high-fat meal compared to under fasted conditions. The change in the Tmax was statistically significant for both memantine and donepezil.

There was no statistically significant difference in total exposure and peak concentration of memantine and donepezil following administration of an intact MDX-8704 (memantine ER/donepezil) 28 mg/10 mg capsule versus the capsule contents sprinkled on applesauce under fasted conditions, suggesting the 2 treatments are bioequivalent. The median Tmax of memantine was reduced to 14 hours from 24 hours and the Tmax of donepezil was reduced to 2.1 hours from 3.0 hours when administered as an intact capsule under fasted conditions versus as capsule contents sprinkled on applesauce under fasted conditions. The change in the Tmax was statistically significant for memantine, but was not statistically significant for donepezil.

The incidence of TEAEs was similar for MDX-8704 administered under fasted conditions either as an intact capsule or as capsule contents sprinkled on applesauce and lower for MDX-8704 administered after a high-fat meal.

Clinically significant safety signals were not observed in this study. A single oral dose of MDX-8704 (memantine ER/donepezil) 28 mg/10 mg capsule was generally safe and tolerable in the healthy male and female subjects in this study.

## Claims

1. Oral dosage form comprising a composition comprising donepezil or a pharmaceutically acceptable salt thereof for use in a method of treating moderate to severe dementia of the Alzheimer's type, wherein the composition has an angle of repose of less than 40 degrees; wherein the dosage form comprises memantine or a pharmaceutically acceptable salt thereof; and wherein more than 60% of the donepezil or pharmaceutically acceptable salt thereof dissolves within 5 minutes of administration to the patient.

2. Oral dosage form comprising a composition comprising donepezil or a pharmaceutically acceptable salt thereof for use in a method of treating moderate to severe dementia of the Alzheimer's type, wherein the dosage form may be sprinkled on food and more than 80% of the donepezil or pharmaceutically acceptable salt thereof dissolves within 30 minutes of administration to the patient; wherein the dosage form comprises memantine or a pharmaceutically acceptable salt thereof.

3. Oral dosage form comprising a composition comprising donepezil or a pharmaceutically acceptable salt thereof for use in a method of treating moderate to severe dementia of the Alzheimer's type, wherein the dosage form has a volume of less than 0.70 ml and the composition has a drug loading of 10%; wherein the composition comprises 10 mg donepezil or a pharmaceutically acceptable salt thereof; wherein the dosage form comprises memantine or a pharmaceutically acceptable salt thereof; and wherein more than 60% of the donepezil or pharmaceutically acceptable salt thereof dissolves within 5 minutes of administration to the patient.

4. The oral dosage form for use according to claim 1, wherein the composition comprises 10 mg donepezil or a pharmaceutically acceptable salt thereof.

5. The oral dosage form for use according to claim 1, 2 or 3, wherein the dosage form comprises 28 mg memantine or a pharmaceutically acceptable salt thereof.

6. The oral dosage form for use according to claim 2 or 3, wherein the composition has an angle of repose of less than 40 degrees.

7. The oral dosage form for use according to claim 1, 2 or 3, wherein the angle of repose is less than 35 degrees, preferably less than 30 degrees.

8. The oral dosage form for use according to claim 1, 2 or 3, wherein the angle of repose is between 25 to 40 degrees.

9. The oral dosage form for use according to claim 1 or3, wherein the dosage form may be sprinkled on food and more than 80% of the donepezil or pharmaceutically acceptable salt thereof dissolves within 30 minutes of administration to the patient.

10. The oral dosage form for use according to claim 1, 2 or 3, wherein more than 60%, preferably more than 70% of the donepezil or pharmaceutically acceptable salt thereof dissolves within 5 minutes of administration to the patient.

11. The oral dosage form for use according to claim 1, wherein more than 20%, preferably more than 30% of the donepezil or pharmaceutically acceptable salt thereof dissolves within 2.5 minutes of administration to the patient.

12. The oral dosage form for use according to claim 1 or2, wherein the dosage form has a volume of less than 0.70 ml, less than 0.50 ml and preferably less than 0.40 ml.

13. The oral dosage form for use according to claim 1 or2, wherein the composition has a drug loading of 10%.

14. The oral dosage form for use according to claim 3, wherein the dosage form has a volume of less than 0.50 ml or less than 0.40 ml and the composition has a drug loading of 10%.

15. Donepezil HCl granular composition comprising an intragranular component and an extragranular component; wherein said intragranular component comprises 10% donepezil HCl, 60% lactose monohydrate, 22% microcrystalline cellulose, 7.2% corn starch, 0.3% magnesium stearate, 0.1% colloidal silicon dioxide; and wherein said extragranular component comprises 0.2% colloidal silicon dioxide and 0.2% magnesium stearate, wherein the percentages are relative to the granular composition.

## Patentansprüche

1. Orale Darreichungsform, umfassend eine Zusammensetzung, umfassend Donepezil oder ein pharmazeutisch annehmbares Salz davon, zur Verwendung in einem Verfahren zur Behandlung von mittelschwerer bis schwerer Demenz vom Alzheimertyp, wobei die Zusammensetzung einen Schüttwinkel von weniger als 40 Grad aufweist, wobei die Darreichungsform Memantin oder ein pharmazeutisch annehmbares Salz davon umfasst und wobei sich mehr als 60 % des Donepezils oder pharmazeutisch annehmbaren Salzes davon innerhalb von 5 Minuten nach Verabreichung an den Patienten auflösen.

2. Orale Darreichungsform, umfassend eine Zusammensetzung, umfassend Donepezil oder ein pharmazeutisch annehmbares Salz davon, zur Verwendung in einem Verfahren zur Behandlung von mittelschwerer bis schwerer Demenz vom Alzheimertyp, wobei die Darreichungsform über ein Nahrungsmittel gestreut werden kann und sich mehr als 80 % des Donepezils oder pharmazeutisch annehmbaren Salzes davon innerhalb von 30 Minuten nach Verabreichung an den Patienten auflösen, wobei die Darreichungsform Memantin oder ein pharmazeutisch annehmbares Salz davon umfasst.

3. Orale Darreichungsform, umfassend eine Zusammensetzung, umfassend Donepezil oder ein pharmazeutisch annehmbares Salz davon, zur Verwendung in einem Verfahren zur Behandlung von mittelschwerer bis schwerer Demenz vom Alzheimertyp, wobei die Darreichungsform ein Volumen von weniger als 0,70 ml aufweist und die Zusammensetzung eine Wirkstoffbeladung von 10 % aufweist, wobei die Zusammensetzung 10 mg Donepezil oder ein pharmazeutisch annehmbares Salz davon umfasst, wobei die Darreichungsform Memantin oder ein pharmazeutisch annehmbares Salz davon umfasst und wobei sich mehr als 60 % des Donepezils oder pharmazeutisch annehmbaren Salzes davon innerhalb von 5 Minuten nach Verabreichung an den Patienten auflösen.

4. Orale Darreichungsform zur Verwendung gemäß Anspruch 1, wobei die Zusammensetzung 10 mg Donepezil oder eines pharmazeutisch annehmbaren Salzes davon umfasst.

5. Orale Darreichungsform zur Verwendung gemäß Anspruch 1, 2 oder 3, wobei die Darreichungsform 28 mg Memantin oder eines pharmazeutisch annehmbaren Salzes davon umfasst.

6. Orale Darreichungsform zur Verwendung gemäß Anspruch 2 oder 3, wobei die Zusammensetzung einen Schüttwinkel von kleiner als 40 Grad aufweist.

7. Orale Darreichungsform zur Verwendung gemäß Anspruch 1, 2 oder 3, wobei der Schüttwinkel weniger als 35 Grad, vorzugsweise weniger als 30 Grad, beträgt.

8. Orale Darreichungsform zur Verwendung gemäß Anspruch 1, 2 oder 3, wobei der Schüttwinkel zwischen 25 und 40 Grad beträgt.

9. Orale Darreichungsform zur Verwendung gemäß Anspruch 1 oder 3, wobei die Darreichungsform über ein Nahrungsmittel gestreut werden kann und sich mehr als 80 % des Donepezils oder pharmazeutisch annehmbaren Salzes davon innerhalb von 30 Minuten nach Verabreichung an den Patienten auflösen.

10. Orale Darreichungsform zur Verwendung gemäß Anspruch 1, 2 oder 3, wobei sich mehr als 60 %, vorzugsweise mehr als 70 %, des Donepezil oder des pharmazeutisch annehmbaren Salzes davon innerhalb von 5 Minuten nach Verabreichung an den Patienten auflösen.

11. Orale Darreichungsform zur Verwendung gemäß Anspruch 1, wobei sich mehr als 20 %, vorzugsweise mehr als 30 %, des Donepezil oder pharmazeutisch annehmbaren Salzes davon innerhalb von 2,5 Minuten nach Verabreichung an den Patienten auflösen.

12. Orale Darreichungsform zur Verwendung gemäß Anspruch 1 oder 2, wobei die Darreichungsform ein Volumen von weniger als 0,70 ml, weniger als 0,50 ml und vorzugsweise weniger als 0,40 ml aufweist.

13. Orale Darreichungsform zur Verwendung gemäß Anspruch 1 oder 2, wobei die Zusammensetzung eine Wirkstoffbeladung von 10 % aufweist.

14. Orale Darreichungsform zur Verwendung gemäß Anspruch 3, wobei die Darreichungsform ein Volumen von weniger als 0,50 ml oder weniger als 0,40 ml aufweist und die Zusammensetzung eine Wirkstoffbeladung von 10 % aufweist.

15. Granuläre Donepezil-HCl-Zusammensetzung, umfassend eine intragranuläre Komponente und eine extragranuläre Komponente, wobei die intragranuläre Komponente 10 % Donepezil-HCl, 60 % Lactosemonohydrat, 22 % mikrokristalline Cellulose 7,2 % Maisstärke, 0,3 % Magnesiumstearat, 0,1 % kolloidales Siliciumdioxid umfasst und die extragranuläre Komponente 0,2 % kolloidales Siliciumdioxid und 0,2 % Magnesiumstearat umfasst, wobei sich die Prozentanteile auf die granuläre Zusammensetzung beziehen.

## Revendications

1. Forme posologique orale comprenant une composition comprenant du donépézil ou un sel pharmaceutiquement acceptable de celui-ci pour une utilisation dans un procédé de traitement de la démence modérée à sévère de type Alzheimer, dans laquelle la composition a un angle de repos inférieur à 40 degrés; dans laquelle la forme posologique comprend de la mémantine ou un sel pharmaceutiquement acceptable de celle-ci; et dans laquelle plus de 60 % du donépézil ou du sel pharmaceutiquement acceptable de celui-ci se dissout dans les 5 minutes suivant l'administration au patient.

2. Forme posologique orale comprenant une composition comprenant du donépézil ou un sel pharmaceutiquement acceptable de celui-ci pour une utilisation dans un procédé de traitement de la démence modérée à sévère de type Alzheimer, dans laquelle la forme posologique peut être saupoudrée sur des aliments et plus de 80 % du donépézil ou du sel pharmaceutiquement acceptable de celui-ci se dissout dans les 30 minutes suivant l'administration au patient ; dans laquelle la forme posologique comprend de la mémantine ou un sel pharmaceutiquement acceptable de celle-ci.

3. Forme posologique orale comprenant une composition comprenant du donépézil ou un sel pharmaceutiquement acceptable de celui-ci pour une utilisation dans un procédé de traitement de la démence modérée à sévère de type Alzheimer, dans laquelle la forme posologique a un volume inférieur à 0,70 ml et la composition a une charge en médicament de 10 % ; dans laquelle la composition comprend 10 mg de donépézil ou un sel pharmaceutiquement acceptable de celui-ci ; dans laquelle la forme posologique comprend de la mémantine ou un sel pharmaceutiquement acceptable de celle-ci ; et dans laquelle plus de 60 % du donépézil ou du sel pharmaceutiquement acceptable de celui-ci se dissout dans les 5 minutes suivant l'administration au patient.

4. Forme posologique orale pour une utilisation selon la revendication 1, dans laquelle la composition comprend 10 mg de donépézil ou un sel pharmaceutiquement acceptable de celui-ci.

5. Forme posologique orale pour une utilisation selon la revendication 1, 2 ou 3, dans laquelle la forme posologique comprend 28 mg de mémantine ou un sel pharmaceutiquement acceptable de celui-ci.

6. Forme posologique orale pour une utilisation selon la revendication 2 ou 3, dans laquelle la composition a un angle de repos inférieur à 40 degrés.

7. Forme posologique orale pour une utilisation selon la revendication 1, 2 ou 3, dans laquelle l'angle de repos est inférieur à 35 degrés, de préférence inférieur à 30 degrés.

8. Forme posologique orale pour une utilisation selon la revendication 1, 2 ou 3, dans laquelle l'angle de repos est compris entre 25 et 40 degrés.

9. Forme posologique orale pour une utilisation selon la revendication 1 ou 3, dans laquelle la forme posologique peut être saupoudrée sur des aliments et plus de 80 % du donépézil ou du sel pharmaceutiquement acceptable de celui-ci se dissout dans les 30 minutes suivant l'administration au patient.

10. Forme posologique orale pour une utilisation selon la revendication 1, 2 ou 3, dans laquelle plus de 60 %, de préférence plus de 70 %, du donépézil ou du sel pharmaceutiquement acceptable de celui-ci se dissout dans les 5 minutes suivant l'administration au patient.

11. Forme posologique orale pour une utilisation selon la revendication 1, dans laquelle plus de 20 %, de préférence plus de 30 %, du donépézil ou du sel pharmaceutiquement acceptable de celui-ci se dissout dans les 2,5 minutes suivant l'administration au patient.

12. Forme posologique orale pour une utilisation selon la revendication 1 ou 2, dans laquelle la forme posologique a un volume inférieur à 0,70 ml, inférieur à 0,50 ml et de préférence inférieur à 0,40 ml.

13. Forme posologique orale pour une utilisation selon la revendication 1 ou 2, dans laquelle la composition a une charge en médicament de 10 %.

14. Forme posologique orale pour une utilisation selon la revendication 3, dans laquelle la forme posologique a un volume inférieur à 0,50 ml ou inférieur à 0,40 ml et la composition a une charge en médicament de 10 %.

15. Composition granulaire de donépézil HCl comprenant un composant intragranulaire et un composant extragranulaire ; dans laquelle ledit composant intragranulaire comprend 10 % de donépézil HCl, 60 % de lactose monohydraté, 22 % de cellulose microcristalline, 7,2 % d'amidon de maïs, 0,3 % de stéarate de magnésium, 0,1 % de dioxyde de silicium colloïdal ; et dans laquelle ledit composant extragranulaire comprend 0,2 % de dioxyde de silicium colloïdal et 0,2 % de stéarate de magnésium, dans lequel les pourcentages sont relatifs à la composition granulaire.
